# EUROPEAN PATENT APPLICATION

(11) **EP 3 189 775 A1**
(43) Date of publication of application: **12.07.2017**
(21) Application number: 16150224.0
(22) Date of filing: 05.01.2016
(51) Int. Cl.: A61B 5/00, A61B 5/053

(54) **BIOSENSOR FOR MONITORING BONE HEALING**

(71) Applicant: Lepperdinger, Günter, 5112 Lamprechtshausen (AT)
(72) Inventor: LEPPERDINGER, Günter, 5112 Lamprechtshausen (AT); ERTL, Peter, 1190 Vienna (AT); GASSNER, Robert, 6020 Innsbruck (AT)
(74) Representative: Gassner, Birgitta

(57) **Abstract**

Endosseous implants are routinely applied in traumatology to support tissue reconstruction and bone healing. A major concern is weak and failing implant integration. Impaired healing leads to severe patient suffering and ultimately requires revision surgery. Only an early diagnosis at the implant surface allows timely therapeutic intervention of impaired osseous healing.

## Description

### Field of the Invention

The present invention relates to the field of portable sensors, particularly implantable biosensor chips for monitoring tissue formation in a patient. The implantable sensor chip comprises a substrate layer and two or more interwoven impedance sensor layouts. The present invention relates as well to continuous monitoring of hard and soft tissue formation after implantation of an endoprostheses or osteosyntheses device.

### Background Art

In Western societies the steady increase in human life expectancy is posing a significant socioeconomic challenge to maintain existing medical therapy standards and health care for senior patients. Among others, falls-related injuries represent a major medical and financial risk factor for older people and their families, where even low trauma fractures considerably induce long-term mortality. The higher incident of fall-related injuries in combination with diminished recovery rates in elderly patients not only leads to prolonged patient suffering, but also results in the inability to cope with daily routines, loss of independence and the need for personal care and assistance. In many cases the loss of independence is also accompanied with confusion and depression, also called "post fall syndrome", which further restricts daily activities. Therefore endoprosthetic surgery is often performed which allows early mobilization and weight bearing ability after traumatic episodes. In addition to internal fixations using osteosyntheses, endoprostheses made of titanium alloy are commonly implanted to replace entire joints (e.g. hip, knee etc.). Despite its routine clinical application endoprosthetic fixation still remains a race between implant failure and patient's death. Besides complicated cases of tumor progression necessitating revision surgery of implanted endoprostheses, it is well known that elderly patients exhibit a significantly higher risk of complications which often is due to aseptic loosening, infections and structural defects.

To prevent postoperative and long-term mechanical complications, an important strategy of medical traumatology involves repeated follow-up examinations using a variety of *in vivo* imaging technologies such as X-ray, dual-energy X-ray absorptiometry, and scintigraphy. The major limitation of these *in vivo* imaging technologies, however, is the accumulation of harmful radiation doses that may pose a risk to the patient's health. Another drawback of existing *in vivo* diagnostic methods is their limited resolution that prevents, both in the context of fracture healing or after implant integration, the identification of the onset and rate of osseous repair. It is important to note that only an early prognostic assessment of implant integration and bone healing will allow a timely and successful pharmacological intervention and adjustment of adequate adjuvant therapies, which help to eliminate further complications. To improve early diagnosis a variety of sensory systems suitable for implantology have been developed to accurately predict implant stability by assessing mechanical forces, oxygen tension, temperature fluctuations and electrochemical parameters during healing processes. Although the determination of mechanical stability is essential for the assessment of long-term performance of joint replacements, no standardized pharmacological and therapeutic recommendations based on implant stability measures could be established so far.

To overcome this methodological divide, a non-invasive and prognostic imaging technology capable of monitoring the dynamics of bone formation at the implant surface and its adjacent vicinity is needed.

### Summary of invention

It is the objective of the present invention to provide an implantable biosensor based on contactless impedance spectroscopy which is capable of 3D-detection of the onset and progression of tissue formation directly at the tissue implant bio-interface and its adjacent vicinity.

The objective is solved by the subject of the present invention.

According to the invention there are provided novel impedance sensors geometry, and methods for sensing and monitoring tissue and/or bone formation in a patient after receiving an implanted device.

According to the invention there is provided a biosensor comprising a substrate layer, at least two interwoven impedance sensor layouts, optionally covered by a metal oxide layer for insulating the biosensor surface, and an electronic read out unit.

According to the invention there is provided the use of such a biosensor for monitoring tissue remodeling and/or bone repair.

According to the invention there is provided a method for monitoring tissue formation in a patient comprising the steps of
providing an implantable sensor according to the subject of the invention, providing an external monitoring component;
wirelessly activating the sensor disposed in a tissue to cause the sensor to sense formation of the tissue; and
transmitting a signal indicative of the sensed tissue formation from the sensor to the external monitoring component.

According to the invention there is provided a kit comprising an [endoprosthesis] and at least one biosensor according to the invention.

### Brief description of drawings

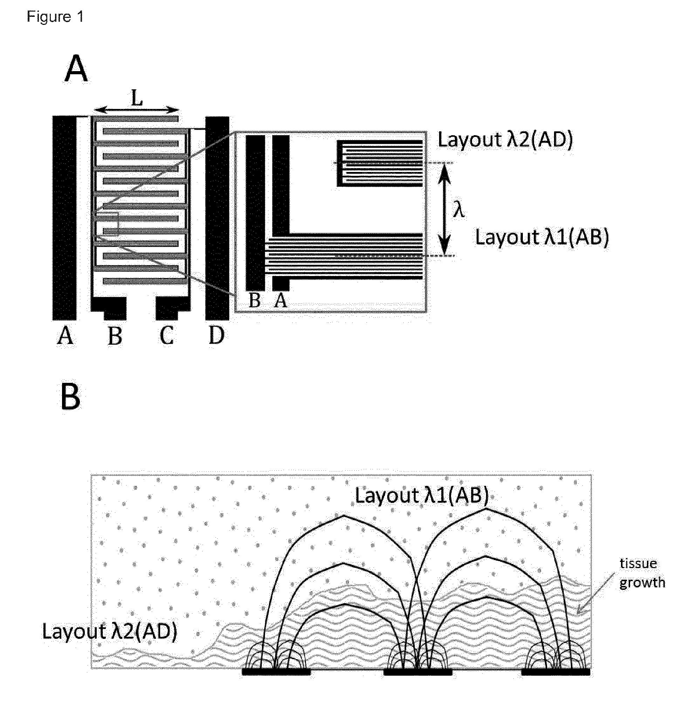

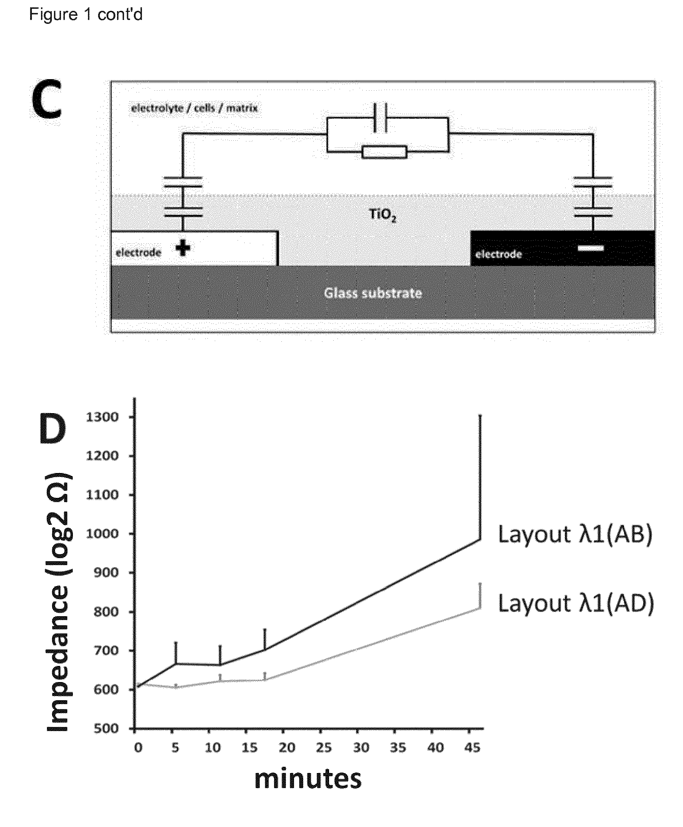

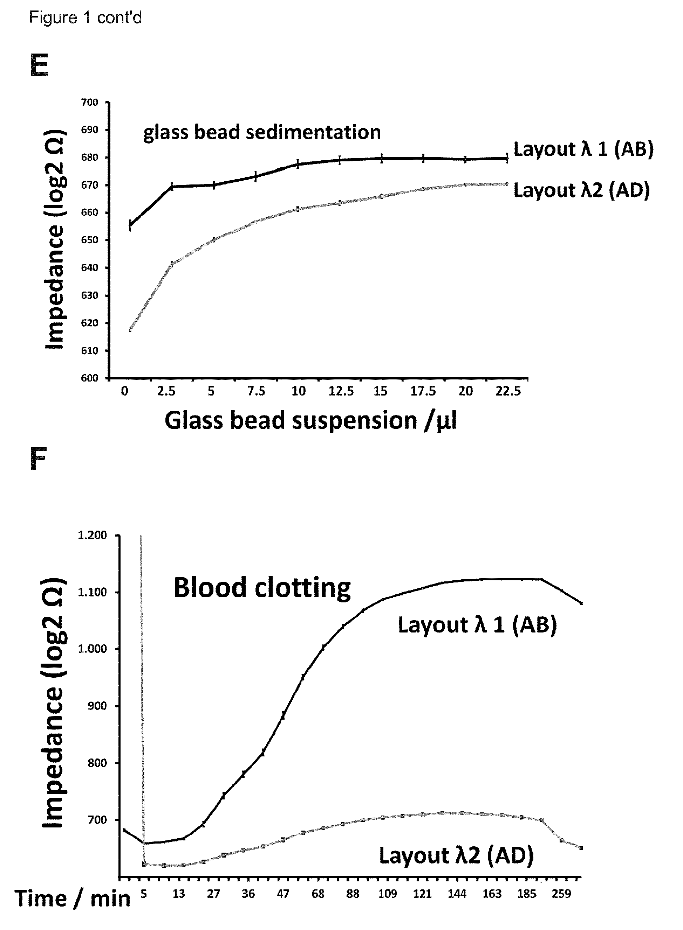

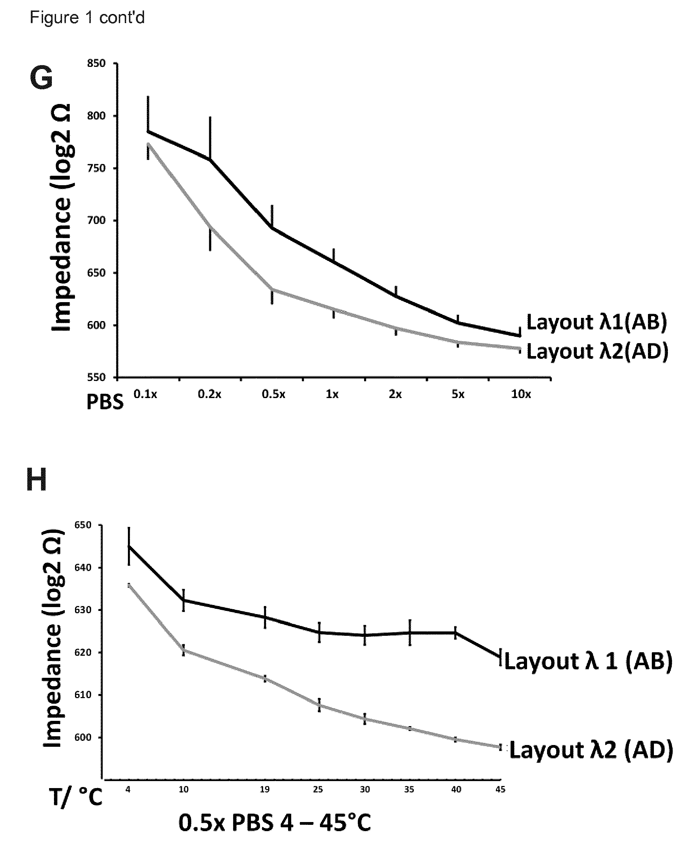

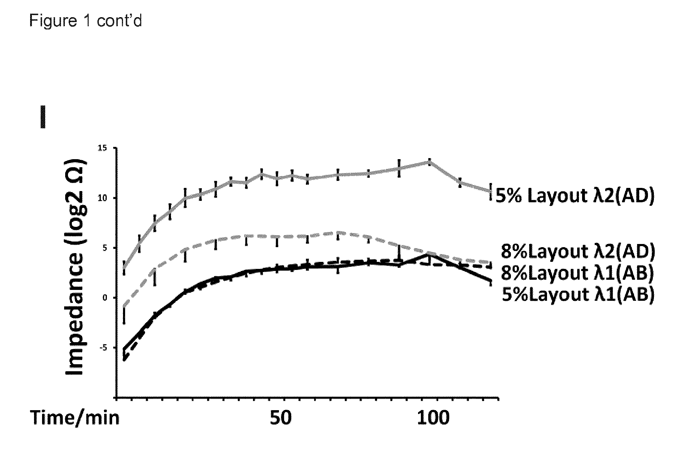

Figure 1. Sensor design and function.
(A) Layout: The monitoring system comprised two sensors with geometries which are significantly differed in the sensing volume, where one sensor layout λ2(AB) can be either independently addressed or also as part of sensor layout λ1(AD) operated. While sensor layout λ1(AD) detects impedance changes directly at the sensor surface, sensor layout λ2(AB) records impedance changes above the sensor surface.
(B) Schematic representation of electric field distribution of the 3D-impedance sensor design
(C) Schematic section of a sensor field with the sensor surface and the respective equivalent circuit model: The interdigitated electrodes (black or white bar) were placed on a glass substrate (dark grey) and were covered by a TiO₂ layer of 50 nm thickness. The capacitance of the TiO₂ layer is in series with the tissue impedance (drawing not to scale). (C-F) Sensograms:
(D) Impedance-time traces of calcium phosphate precipitation in phosphate-buffered saline.
(E) Impedance signals in the presence of increasing concentrations of inorganic bodies such as of glass beads (average diameter of 30 µm) dissolved in 0.5xPBS.
(F) Impedance-time traces of blood coagulation using freshly drawn blood.
(G) The performances of the two sensors were tested in parallel by assessing impedance at varying salt concentrations.
(H) The performances of the two sensors were tested in parallel by assessing impedance at 0.5xPBS at varying temperatures.
(I) The performances of the two sensors were tested in parallel by assessing impedance at 8% or 5% gelatin in 0.5xPBS over time referred to the impedance of 0.5xPBS.

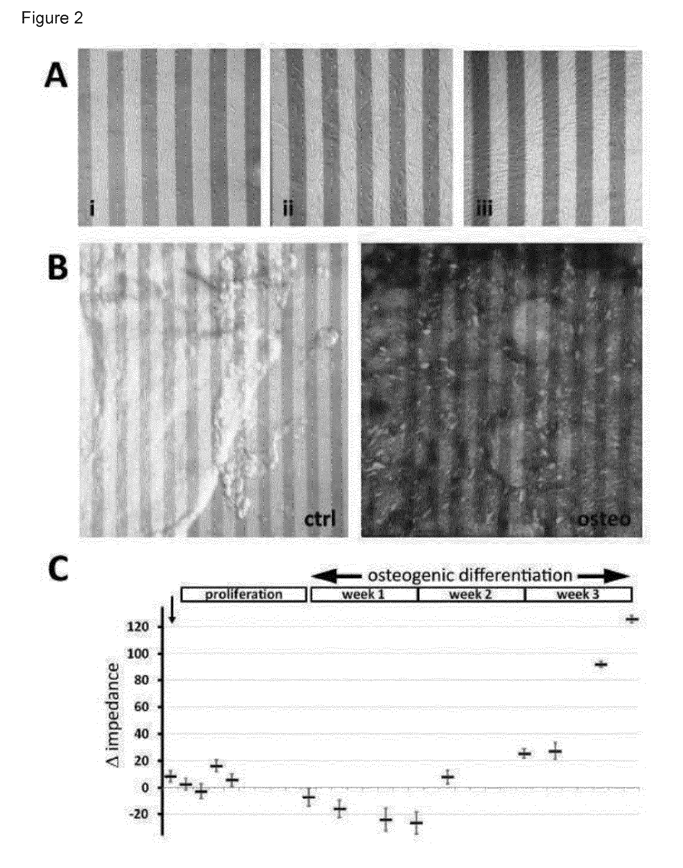

Figure 2. Sensor response to osteogenic differentiation using on-sensor *in vitro* stem cell culture. (A) Microscopic images of human bone-derived mesenchymal stromal cells after (i) seeding, (ii) proliferation under standard culture conditions for several days, and (iii) confluent stage (iii). (B) Images of Alizarin S Red stained osteoblasts (osteo) following on-chip osteogenic differentiation of stromal cells on top of the TiO₂ layer of the sensor surface. (C) Time-resolved impedimetric sensogram of human mesenchymal stromal cells featuring proliferation and osteogenic differentiation over a period of 21 days.

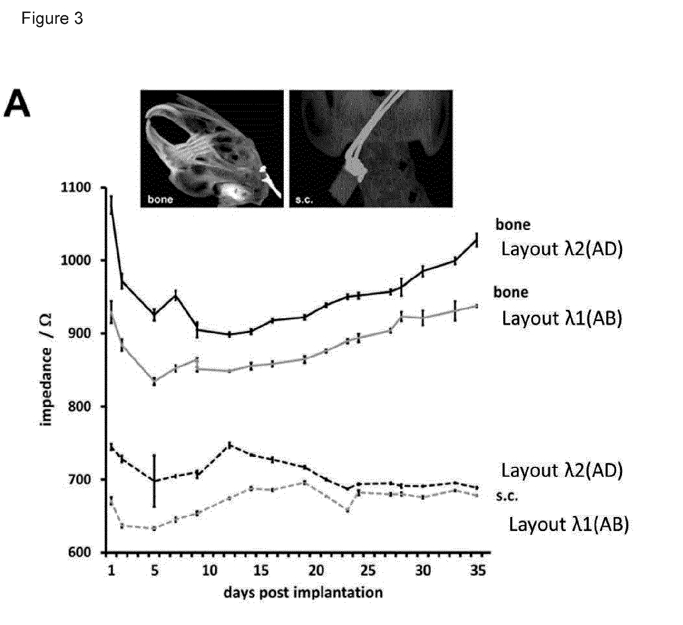

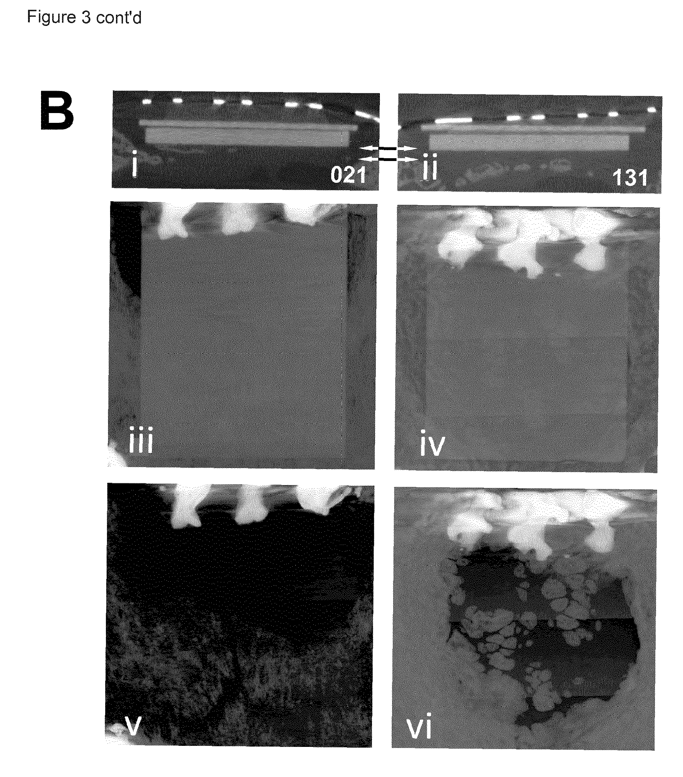

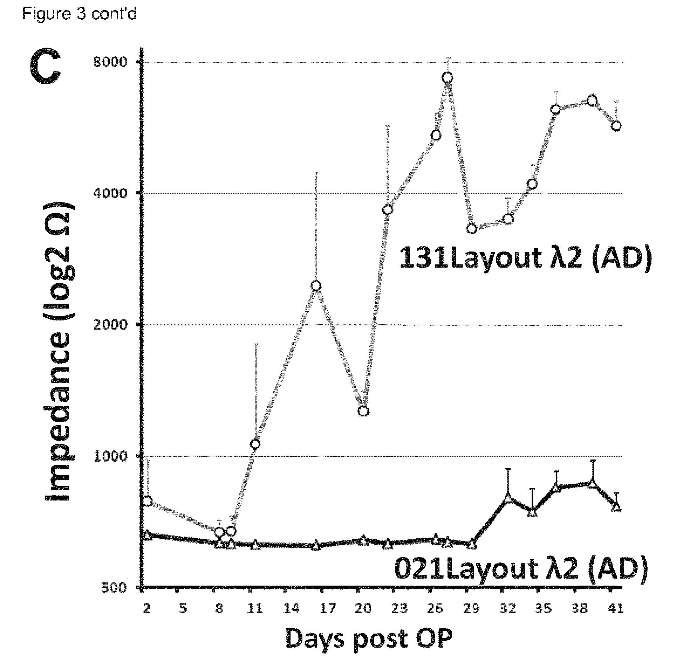

Figure 3. Evaluation of *in vivo* osseous healing.
(A) Time-resolved single frequency (at 99 kHz) impedance signals from both sensor layouts of two biosensors derived from continuous impedance-time spectra recorded from 1 - 99 kHz. Images show position of both biosensors located either in the osseous calvarial defect (bone) or between the skin and the muscle layer in the neck region (s.c.).
(B) Micro-computer-tomographic images of regular and impaired bone formation in experimental rabbit 021 and 131 featuring coronal sections (i, ii) indicating positions of the scans at the sensor surface (shown in iii and iv) and within the defect area (v, vi).
(C) Single frequency impedance-time traces of sensor layout λ1(AB) during 6 weeks of normal and impaired osseous healing observed in rabbits 021 and 131.

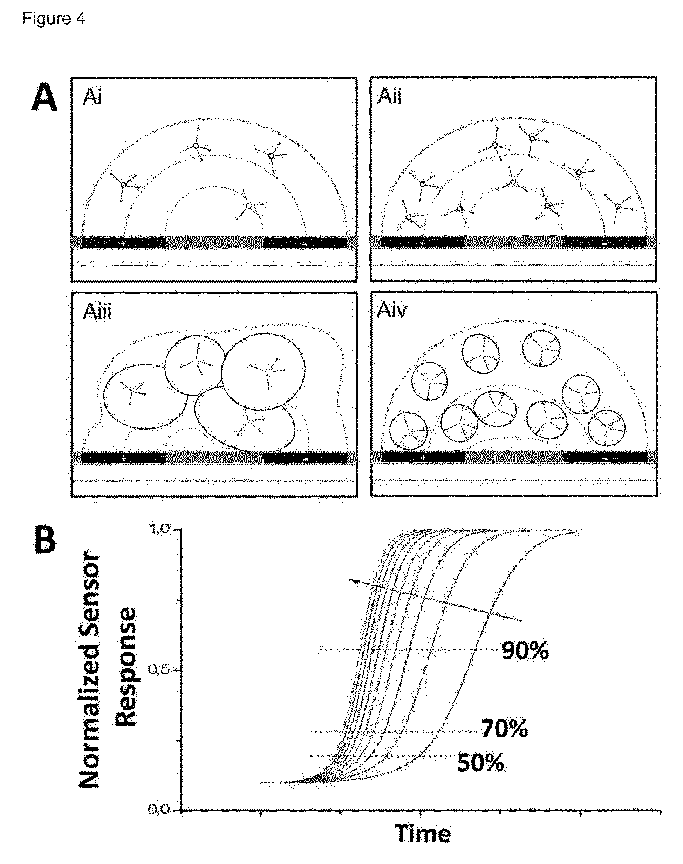

Figure 4. *In silico* modeling of bone healing.
(A) Schematic illustration of non-linear bone formation originating along several nucleation points and corresponding electrical field distributions during osseous regeneration including (i,ii) calcification at nuclear structures and appearance of initiation nuclei; (iii) accumulation and growth of high density infiltrates, and (iv) early bone formation.
(B) Computational estimation of non-linear growth dynamics and bone regeneration rates in the presences of increasing densities of nucleation points. Percentage of the filled sensor volume (Layout λ1(AB)) by the low conductive phase (high density infiltrates) are shown in dashed lines.

### Description of embodiments

The present invention relates to a biosensor and methods for monitoring tissue formation wherein said method is based on contactless 3D impedance sensing (CIS) using stable, non-conducting and biocompatible/ functionalized materials for sensor coating, which are equivalent to or compatible with commonly used implant materials such metal oxides including titanium-, zirconium-, tantalum-oxides and others. CIS is a label-free and non-destructive method that is ideally suited to detect polarization responses of low dielectric materials such as (bio)composites including bone, protein networks and cells within a given sensing volume

CIS has already being employed to monitor growth rates in large-scale liquid suspensions [1,2]. Also microbial cells, viruses, lipid vesicles and human cell cultures *in vitro* have been examined. The main advantage of CIS technology is that it allows the analyses of electrical properties of biological materials perpendicular to the sensor surface with a high degree of spatial resolution. By employing interdigitated electrode structures (IDES) electrical field distribution in z-direction can be controlled by adjusting sensor geometries (see also Figure 1A). Consequently, CIS technology can provide information on dynamic changes of the cellular microenvironment directly adjacent to the sensor surface. Although it is well known that the intrinsic electrical properties of tissues, cells and biofluids can range from insulating to electrically conductive the application of metal oxide-coated microelectrodes to monitor *in vivo* tissue remodeling and repair has not been applied so far.

The aim of the present invention was the early assessment of tissue and/or bone formation *in situ* in order to allow an accurate prediction of implant integration during healing.

In a first aspect there is provided a biosensor comprising a substrate layer, at least two interwoven impedance sensor layouts, optionally covered by a metal oxide layer for insulating the biosensor surface, and an electronic read out unit.

The substrate layer may be made from any suitable material such as glass, silicon, ceramic, non-conducting polymer or a plastic substrate. By non-conducting polymer is meant a polymer with a conductivity of less than 10⁸ S/m.

An impedance sensor layout comprises interdigitated electrodes (IDES) which are composed of two interdigitated electrodes with at least two connection tracks, on a suitable substrate.

An interdigitated configuration typically enhances sensitivity and detection limits of the biosensor, provides defined sensing areas and volumes. The output signal strength of IDES is controlled through careful design of the active area, width, and spacing of the electrode fingers.

Thus, in one embodiment of the invention the impedance sensor layout comprises an IDES.

The electrode width of the two interdigitated electrodes may vary from 1 µm to 250 µm, and the gap between the fingers may vary from 1 µm to 250 µm.

In one embodiment of the invention the interdigitated electrode structure has a ratio between electrode widths to gap of about 1:1 to 1:100, preferably of 1:1 to 1:10.

In one embodiment of the invention, each electrode finger of an impedance sensor layout can independently have a spacing and width in the range from greater than or about 1 µm to about 250 µm, or with a range from greater than or about 10 or 150 µm or about 15 µm.

In one embodiment of the invention the impedance sensor layout comprises at least 50 electrically conductive fingers, preferably at least 70 fingers, more preferred at least 80 fingers.

The total area of the double-interdigitated impedance sensor can be chosen depending on the dimensions of layout λ1 and λ2, on the use intended for the electrodes, on the desired current level intended to pass through the electrodes, and on the desired number of electrode fingers. An exemplary area of a double-interdigitated impedance sensor layout having adequate electrode fingers can be in the range from about 1.0 to about 5 mm², preferably in the range of about 2.0 to 3.5 mm², more preferred of about 2.5 mm². For example an impedance sensor layout having 83 electrode fingers with a spacing and width of 15 µm and a length of 2.02 mm covers an area of 2.5 mm².

In one embodiment of the invention, each electrode finger of the second impedance sensor layout λ2 can independently have a spacing and width in the range from greater than or about 1 µm to about 250 µm, or with a range from greater than or about 10 or 150 µm or about 150 µm.

In one embodiment of the invention the impedance sensor layout λ2comprises at least 5 gold fingers, preferably at least 8 gold fingers, more preferred at least 10 gold fingers.

An exemplary area for an impedance sensor layoutλ2 having adequate electrode fingers can be in the range from about 2.0 to about 10.0 mm², preferably in the range of about 3.0 to 7.5 mm², more preferred of about 6.7 mm². For example an impedance sensor layout (λ2) having 12 electrode fingers with a spacing and width of 150 µm and a length of 3.74 mm covers an area of 6.7 mm².

However, the total area of the impedance sensor layout λ1 and λ2 can be chosen depending on these dimensions, on the use intended for the electrodes, on the desired current level intended to pass through the electrodes, and on the desired number of electrode fingers.

The thickness of the electrode fingers can be sufficient to support a desired electric current. Exemplary thicknesses can be in the range from about 30 to 200 nm, with a preferred thickness being about 50 nm.

In one embodiment of the invention the impedance sensor layout is covered by a non-conducting insulating layer. The insulating layer can be a metal oxide layer, a nitride or polymer layer.

Examples of a non-conductive polymer are a fluoropolymers such as fluoroethylenepropylene (FEP), polytetrafluoroethylene (PFTE, Teflon®), ethylene-tetrafluoroethylene (ETFE), tetrafluoroethylene-perfluoromethyl-vinylether (MFA), vinylidene fluoride-hexafluoropropylene copolymer, tetrafluoroethylene-hexafluoropropylene copolymer, polyvinylidenedifluoride (PVDF), vinylidene fluoride-hexafluoropropylene-tetrafluoroethylene terpolymer, perfluoromethyl vinyl ether-tetrafluoro-ethylen copolymer, perfluoroalkoxy copolymer (PFA), poly(vinyl fluoride), polychlorotrifluoroethylene, a fluorosilicone or a fluorophosphazene. Further examples of a nonconductive polymer are a polyimide, a polyetherimide, a polyether, a polyester, a polyurethane, a polycarbonate, a polysulfone, and a polyethersulfone.

Examples of a non-conductive nitride polymer are silicon nitride, oxidenitride, or the like:

In one embodiment of the invention the insulating layer is a TiO₂ or ZrOx layer.

The insulating layer can have a thickness of less than 1 µm, preferably of less than 100 nm, more preferred of about 50 nm.

The present invention is particularly useful for monitoring soft tissue formation and/or ossifying material in a patient after implant surgery.

Endosseous implants are routinely applied in traumatology to support tissue reconstruction and bone healing. A major concern is weak and failing implant integration. Impaired healing leads to severe patient suffering and ultimately needs revision surgery. Only an early diagnosis at the implant surface allows timely therapeutic intervention of impaired osseous healing

Thus, a further embodiment of the invention relates to the use of such a biosensor for monitoring tissue remodeling and/or bone repair.

One embodiment of the invention relates to an *ex vivo* method for monitoring tissue formation in a patient comprising the steps of:
a. providing an implantable sensor of the present invention, providing an external monitoring component;
b. wirelessly activating the sensor grafted in a tissue to cause the sensor to sense formation of the tissue; and
c. transmitting a signal indicative of the sensed tissue formation from the sensor to the external monitoring component.

In one embodiment of the invention the differences between granulation and soft tissue formation are sensed.

In a further embodiment of the invention the formation of ossifying material is sensed.

In a further embodiment of the invention the tissue formation after implantation of an implantable device is sensed. The implantable device according to the invention is a device to be implanted between bony, cartilaginous or soft tissues or between prosthetic surfaces to restore or create a gap. The implantable device can be an endoprosthesis, an osteosyntheses and/or dental implant.

In one embodiment of the invention the endoprostheses is a hip joint or knee endoprosthesis and/or a osteosyntheses for fixation and healing of bone, cartilage and tendon used in plastic and/or orthopedic surgery and/or a dental implant.

In one embodiment of the invention the use of biosensor as defined above for implantation into a human or animal body is encompassed.

One embodiment of the invention relates to a kit comprising an implantable device and at least one biosensor as described herein.

In one embodiment of the invention the implantable device is an endoprosthesis, preferably a hip joint or knee endoprosthesis and/or an osteosynthesis for fixation and healing of bone, cartilage and tendon used in plastic and/or orthopedic surgery and/or a dental implant.

In one embodiment of the invention the biosensor is used for monitoring the stability and endurance of endoprosthesis, osteosynthesis for fixation and healing of bone, cartilage and tendon used in plastic and orthopedic surgery and/or dental implant

In order to proof the concept, titanium-oxide coated IDES were implanted into subcritical defects of rabbit calvaria. Bone is a mineralized matrix consisting of cells embedded in the organic component collagen interspersed with other extracellular proteins that are specific for osteogenic matrix, and, an inorganic mineral component in the form of rod-shaped calcium phosphate crystals. During early stages of bone healing an inhomogeneous anisotropic composite is formed which eventually is undergoing material transformation and structural changes. With (a) the formation of more rigid structures (b) the reduction in water content of a tissue and (c) with an increasing amount of collagenous fibers the electrical resistance of body tissues increases. Therefore anisotropic changes associated with bone healing are detectable with the aid of CIS technology. Indeed, shifts in relative permittivity in composite polymers, which exhibit similar dielectric parameters as bone, can be used to identify structural characteristics and density heterogeneities at the micrometer scale. In fact, the tensile strength of bone has already been correlated to its electrical behavior using cadaveric human trabecular bone samples harvested from the distal femur and proximal tibia.

We here demonstrate the practical application of CIS technology for *in vivo* monitoring of bone healing and implant integration. The current CIS system design features two different IDES sensor geometries coated with an electrically insulating layer of TiO₂, which not only eliminates undesirable interferences including pH changes and electrode polarization events as well as unwanted side effect such as the generation of toxic products by electrochemical reactions, but also closely resembles the material biointerface of the commonly used endosseous titanium implants. As a result, the developed contactless biosensor technology can ultimately be integrated into any endoprosthetic implant to detect the formation of granulation tissue. This means that accurate identification of soft to hard tissue conversion will be possible during the initial healing stages, thus allowing the prediction on the efficacy of osseous repair rate.

### Examples

The Examples which follow are set forth to aid in the understanding of the invention but are not intended to, and should not be construed to limit the scope of the invention in any way. The Examples do not include detailed descriptions of conventional methods which are well known to those of ordinary skill in the art.

### Example 1: Sensor responses to critical biological parameter

Key features of the contactless biosensor system include the integration of two independently addressable interwoven IDES geometries with an insulating 50-nm TiO₂ layer for complete sensor coverage. Figure 1A shows a schematic layout of the impedimetric sensor designs featuring electrode areas of approximately 2.5 mm² (λ2) and 6.7 mm² (λ1) that contain width-to-gap-ratios of 150 µm (λ1) and 15 µm (λ2), respectively. The applied electrode finger width-to-gap-ratios were selected to produce electric field distributions that are able to reach increasing distances from the sensor surface. In the present configuration, sensor sensitivities of λ1 and λ2 are limited to 10 µm or 100 µm in z-direction. In other words capacitive signals between one and ten cell layers of the tissue can be probed adjacent to the CIS surface as shown in the simplified impedance model in Fig. 1B and 1C. The sensor was characterized using cyclic voltammetry in the presence of the electro active redox couple ferro-ferricyanide revealing that the TiO₂ layer acts as an efficient insulator by eliminating electrochemical reactions and charge transfer reactions at the gold electrodes. The biosensor was tested in a series of *in vitro* experiments using varying salt concentrations and temperature fluctuations calcium phosphate crystal formation, glass bead sedimentation and blood clotting to assess the impact of putative tissue responses on sensor signal. While increasing salt concentrations and temperature showed only small impedance changes (see Fig. 1 G, 1H), the precipitation of calcium phosphate crystals on top of the TiO₂-coated sensors exhibited a significant impedance increase over time (Fig. 1 D). To experimentally simulate the formation of soft tissue *in vitro,* glass beads with an average diameter of 30±10 µm were piled up onto the sensor surface. Results in Figure 1 E revealed an exponential impedance increase, thus indicating that the formation and growth of non-conductive granules can be detected by the biosensor. Also the coagulation of freshly drawn human blood yielded a considerable impedance increase (Fig. 1 F). The presence of gelatin (0.5% to 8%) resulted in insignificant impedance signal changes over time (Fig. 1H).

To characterize cellular attachment, proliferation and differentiation, human bone-derived mesenchymal stromal cells (MSC) were seeded onto the TiO₂-coated sensor. Fig. 2A shows a series of pictures taken over a period of several days demonstrating that bone precursor-forming MSC firmly attached and proliferated at the TiO₂ covered sensor surface. Successive long-term cultivations revealed proliferation rates comparable to cells grown in commercial plastic dishes. To further verify the absence of electrical field induced side effects on osteoprogenitor cells, MSC were stimulated to undergo osteogenic differentiation in the presence of a ± 100 mV (Vpp 200 mV) alternating electrical field. Results shown in Fig. 2B revealed similar calcium deposition and formation of osseous-like matrix in the presence of continuously active biosensors, thus suggesting that MSC were largely unperturbed by the applied electric field. Additional long-term experiments using time-resolved measurements during a four-week period revealed a steady impedance increase starting approximately 7 days after the induction of osteogenic differentiation (Fig. 2C).

### Example 2: Sensor implantation and monitoring of osseous healing ex vivo

Next, CIS technology was evaluated in an animal model of osseous healing. Prior to implantation, the biosensor was mounted onto a carrier made of titanium with a biocompatible adhesive. This composite was then inserted into a circular bone defect at the calvaria of a rabbit. It has been shown previously that the critical size bone defect in the rabbit calvaria, in which no contingent osseous healing occurs, is larger than 15 mm [3]. Therefore, a 12-mm defect was expected to allow partial-to-complete bone healing within a period of 6 weeks. Using digital volume tomography, bone regeneration at the operation site was also imaged at a resolution of 110 µm immediately after implantation, four weeks later and following scarification of the experimental animals after 6 weeks. Results revealed that most of the calvarial defects experienced *de novo* bone formation after a period of 6 weeks. Altogether, *ex vivo* imaging data indicated that an implanted titanium-dioxide coated biosensor performs comparable to common medical titanium implants.

To investigate whether the current biosensor is well-suited to *ex vivo* monitor osseous regeneration and healing *in vivo,* time-resolved multifrequency impedance measurements ranging from 1 to 99 kHz were performed by connecting the transcutaneous docking cable to the reader-unit. Initial frequency analysis showed that higher frequencies yielded lowest standard deviations, and therefore, impedance-time traces obtained at 99 kHz were computed for all experimental animals. To further enable discrimination between soft or hard tissue formations two biosensors were implanted into a single rabbit one located at the calvaria bone defect while the other sensor was placed subcutaneously into the neck region. This experimental setup was chosen to concomitantly measure impedance changes of two different tissue types within a single individual (see inset of Fig. 3A). Impedance-time traces of the two sensor exhibited distinctly different impedance readings. While impedance signals of the subcutaneous sensors remained almost unchanged over 35 days, the calvarial sensors displayed significant signal changes. Interestingly, during the first 7 days all of the sensors recorded a steep impedance decrease, which correlates with fluid accumulation around the sensor as a consequence of increased vascular permeability shortly after implantation. Following a signal stabilization period of several days, at the rabbit calvaria impedance signal steadily increased over the remaining test period indicating the formation of granulation tissue. In other words, following bleeding and blood clotting during the subsequent reparative phase of osseous healing, fibroblasts were replaced by hard tissue producing osteogenic progenitor cells within the defected area.

To verify that CIS is able to detect bone growth in the sensing regions, a series of secondary analyses were performed. After the sacrification of the experimental animals six weeks post implantation, the biosensors were explanted together with surrounding bone and the specimens were subjected to µCT imaging (Fig. 3B). µCT results confirmed that in all cases, in which efficient osseous healing occurred, the bone-like material was evenly distributed above the titanium sensor surface. To assess variations in bone formation within the generated defect, µCT imaging data were used to generate successive virtual sections exactly parallel to the sensor surfaces. CIS and µCT methods yielded similar results suggesting that sequential regenerative processes took place in a homogenous fashion. The comparable impedance-time traces of senor λ1 and λ2 (15 µm and 150 µm, respectively) shown in Fig. 3A indicate that the cellular microenvironment directly at the sensor surface (<10 µm) did not significantly differ from conditions found within more distal layers (<100 µm).

Although comparably osseous healing was observed in most of the animals, in rare cases extreme healing dynamics were observed. For instance, µCT images shown in Fig. 3B depict experimental results of rabbit calvariae, which exhibited either excessive or only minute bone formation during the 6-weeks measurement period. While µCT analysis of the coronal sections of rabbit 021 revealed only scarce amounts of newly synthesized bone (Fig. 3Bv), radiology of rabbit 131 showed excessive bone nodule formation within the defected area (Fig. 4Bvi). It is conceivable that due to fabrication imperfections traces of conductive silver solder leached through the polyacryl encapsulation, thus inhibiting bone formation at the TiO₂ biointerface. Corresponding impedance-time traces of rabbit 021 exhibited only a marginal impedance increase during the first four weeks, while already 11 days post implantation impedance signals significantly rose in rabbit 131 (Fig. 3C). These observations were also in good agreement with terminal µCT image analysis and the digital volume tomography (DVT) follow-up control performed after four weeks and six weeks post implantation. These results point at the ability of the TiO₂-coated contactless biosensors being able of rapidly identifying the onset and rate of bone regeneration and healing dynamics.

### Example 3: In silico modeling of in vivo bone healing kinetics

In an attempt to link observed non-linear impedance signals to bone healing kinetics a bone healing model was established based on existing *in vitro* and *in vivo* data. The model shown in Fig. 4A primarily accounts for electrical resistance variations within a two component system consisting of an electrical conductive and a low resistance phase. A comparable model has been applied to study the formation of inorganic composites with non-linear growth kinetics and increasing nucleation points [4]. Fig. 4A in particular highlights the initial stages of osseous regeneration in the vicinity of the TiO₂ biointerface where (i) and (ii) show the electrical field distribution during the onset of calcification at randomly located initiation nuclei while (iii) and (iv) depict rapidly growing and regular bone formation. Fig. 4B estimates the effects of nucleation points on dynamic and non-linear bone formation using five parameters thereby representing the current measurement conditions including (i) IDES geometry factor, (ii) inherent resistance of the reader unit, (iii) a geometric factor of non-conducting infiltrates, and (iv, v) the conductivities of blood and bone (σh, σl) The computational results suggest that the applied electrical field is mainly influenced by the emergence of non-conductive geometric elements that sprouted and expanded over time within a milieu of specific resistance.

### References

[1] Justice, C., et al. Process control in cell culture technology using dielectric spectroscopy. Biotechnol Adv 29, 391-401 (2011).
[2] Palmer, S.M. & Kunji, E.R. Online monitoring of biomass accumulation in recombinant yeast cultures. Methods Mol Biol 866, 165-179 (2012).
[3] Schmitz, J.P. & Hollinger, J.O. The critical size defect as an experimental model for craniomandibulofacial nonunions. Clin Orthop Relat Res, 299-308 (1986).
[4] Wang CX & Wang M. Electrochemical impedance spectroscopy study of the nucleation and growth of apatite on chemically treated pure titanium Materials Letters 54:30-36 (2002)

## Claims

1. A biosensor comprising a substrate layer, at least two interwoven impedance sensor layouts, optionally covered by a non-conducting layer for insulating the biosensor surface, and an electronic read out unit.

2. The biosensor according to claim 1, wherein each impedance sensor layout comprises two interdigitated electrode structures (IDES).

3. The biosensor according to claim 1 or 2, wherein said interdigitated electrode structure has a ratio between electrode width to gap of about 1:1 to 1:100, preferably of 1:1 to 1:10.

4. The biosensor according to any one of claims 1 to 3, wherein said substrate layer is a glass, ceramic or polymer layer.

5. The biosensor according to any one of claims 1 to 4, wherein said non-conducting layer is a metal oxide layer, a nitride, or a polymer layer, with a thickness of less than 1 µm, preferably of less than 100 nm, more preferred of about 50 nm.

6. A method for monitoring tissue formation in a patient comprising the steps of providing an implantable sensor according to any one of claims 1 to 5, providing an external monitoring component;
wirelessly activating the sensor grafted in a tissue to cause the sensor to sense formation of the tissue; and
transmitting a signal indicative of the sensed tissue formation from the sensor to the external monitoring component.

7. The method according to claim 6, wherein differences between granulation and soft tissue formation are sensed.

8. The method according to claim 6 or 7, wherein formation of ossifying material is sensed.

9. The method according to claims 6 or 7, wherein said tissue formation is sensed after implantation of an implantable device.

10. The method according to any one claims 6 to 9, wherein tissue formation is sensed at the sensor tissue interface along a vertical axis into the tissue.

11. Use of a biosensor according to any one of claims 1 to 5 for monitoring tissue remodeling and/or bone repair.

12. Use of a biosensor according to any one of claims 1 to 5 for monitoring the stability and endurance of endoprosthesis, osteosynthesis for fixation and healing of bone, cartilage, and tendon used in plastic and orthopedic surgery and /or dental implant.

13. A kit comprising an implantable device and at least one biosensor according to any one of claims 1 to 5.

14. The kit according to claim 13, wherein said implantable device is an endoprosthesis, preferably a hip joint or knee endoprosthesis or an osteosynthesis for fixation and healing of bone, a cartilage, or a tendon used in plastic and/or orthopedic surgery, or a dental implant.
